# EUROPEAN PATENT APPLICATION

(11) **EP 3 882 836 A1**
(43) Date of publication of application: **22.09.2021**
(21) Application number: 21160579.5
(22) Date of filing: 03.03.2021
(51) Int. Cl.: G06Q 10/08, G16H 70/40

(54) **METHOD AND SYSTEM FOR TRACKING DISPENSED AND RETURNED NARCOTICS**

(30) Priority: 17.03.2020 US 202016820965
(71) Applicant: GE Precision Healthcare LLC, Wauwatosa, WI 53226 (US)
(72) Inventor: LAIL, Edward H., Maryville, TN 37801 (US); SHUSTER, Tabatha B., Chattanooga, TN 37405 (US); CAMERON, Kendra C., Knoxville, TN 37932 (US); WHITLEY, Garry M., Clinton, TN 37716 (US); OLMSTEAD, Weston, Greeneville, TN 37743 (US)
(74) Representative: Kilburn & Strode LLP

(57) **Abstract**

A system and method for tracking dispensed and returned narcotics is provided. The method may include receiving a selection of a narcotic bag associated with a narcotic bag electronic record. The method may include retrieving narcotic bag case data from the narcotic bag electronic record associated with the selected narcotic bag. The method may include retrieving an anesthesia narcotic record from electronically stored medical records. The method may include automatically determining expected narcotics to be returned based on the narcotic bag case data and the anesthesia narcotic record. The method may include receiving an indication of actual narcotics returned. The method may include automatically determining whether a discrepancy exists based on the expected narcotics to be returned and the actual narcotics returned. The method may include modifying the narcotic bag electronic record to include at least one of the actual narcotics returned and a discrepancy indicator.

## Description

### FIELD

Certain embodiments relate to tracking narcotics in a medical environment. More specifically, certain embodiments relate to a method and system for tracking dispensed narcotics and calculating unused, returned narcotics based on electronic medical records to detect anesthesia provider diversion and provide analytics related to narcotic use and waste.

### BACKGROUND

Anesthesia providers in a medical environment check-out narcotics to be administered in one or more medical procedures from a pharmacy. The narcotics are administered by an anesthesia provider to patients during the one or more procedures while maintaining an anesthesia narcotic record of the narcotics administered. The anesthesia providers return any unused narcotics to the pharmacy once the one or more procedures are completed. The pharmacy maintains records of the narcotics that are checked out and returned. A pharmacy technician at the pharmacy compares the returned narcotics to the anesthesia narcotic record and the records corresponding to the narcotics that were checked out. The pharmacy technician may document any discrepancies and/or notify an anesthesia supervisor of any discrepancies. This current process of checking out and checking in narcotics is inefficient, prone to error, and is ineffective for detecting anesthesia provider diversion.

Further limitations and disadvantages of conventional and traditional approaches will become apparent to one of skill in the art, through comparison of such systems with some aspects of the present disclosure as set forth in the remainder of the present application with reference to the drawings.

### BRIEF SUMMARY

A system and/or method is provided for tracking dispensed and returned narcotics, substantially as shown in and/or described in connection with at least one of the figures, as set forth more completely in the claims.

These and other advantages, aspects and novel features of the present disclosure, as well as details of an illustrated embodiment thereof, will be more fully understood from the following description and drawings.

### BRIEF DESCRIPTION OF SEVERAL VIEWS OF THE DRAWINGS

FIG. 1 is a block diagram of an exemplary narcotic tracking system that is operable to track dispensed and returned narcotics, in accordance with various embodiments.
FIG. 2 is a flow chart illustrating exemplary steps that may be utilized for checking out narcotics by a narcotic tracking system, in accordance with various embodiments.
FIG. 3 is a flow chart illustrating exemplary steps that may be utilized for checking in narcotics by a narcotic tracking system, in accordance with various embodiments.

### DETAILED DESCRIPTION

Certain embodiments may be found in a method and system for tracking dispensed and returned narcotics. Various embodiments have the technical effect of creating narcotic bag electronic records based on requester information and narcotic check-out information. Aspects of the present disclosure have the technical effect of updating narcotic bag electronic records with narcotic check-in information and discrepancy incidents. Various embodiments have the technical effect of creating and/or updating discrepancy action item worklists to track active recorded discrepancies. Certain embodiments have the technical effect of tracking dispersed and returned narcotics to detect anesthesia provider diversion. Various embodiments have the technical effect of providing analytics related to narcotic use and waste.

The foregoing summary, as well as the following detailed description of certain embodiments will be better understood when read in conjunction with the appended drawings. To the extent that the figures illustrate diagrams of the functional blocks of various embodiments, the functional blocks are not necessarily indicative of the division between hardware circuitry. Thus, for example, one or more of the functional blocks (e.g., processors or memories) may be implemented in a single piece of hardware (e.g., a general-purpose signal processor or a block of random access memory, hard disk, or the like) or multiple pieces of hardware. Similarly, the programs may be stand alone programs, may be incorporated as subroutines in an operating system, may be functions in an installed software package, and the like. It should be understood that the various embodiments are not limited to the arrangements and instrumentality shown in the drawings. It should also be understood that the embodiments may be combined, or that other embodiments may be utilized, and that structural, logical and electrical changes may be made without departing from the scope of the various embodiments. The following detailed description is, therefore, not to be taken in a limiting sense, and the scope of the present disclosure is defined by the appended claims and their equivalents.

As used herein, an element or step recited in the singular and preceded with the word "a" or "an" should be understood as not excluding plural of said elements or steps, unless such exclusion is explicitly stated. Furthermore, references to "an exemplary embodiment," "various embodiments," "certain embodiments," "a representative embodiment," and the like are not intended to be interpreted as excluding the existence of additional embodiments that also incorporate the recited features. Moreover, unless explicitly stated to the contrary, embodiments "comprising", "including", or "having" an element or a plurality of elements having a particular property may include additional elements not having that property.

Furthermore, the term processor or processing unit, as used herein, refers to any type of processing unit that can carry out the required calculations needed for the various embodiments, such as single or multi-core: CPU, Accelerated Processing Unit (APU), Graphic Processing Unit (GPU), DSP, FPGA, ASIC or a combination thereof.

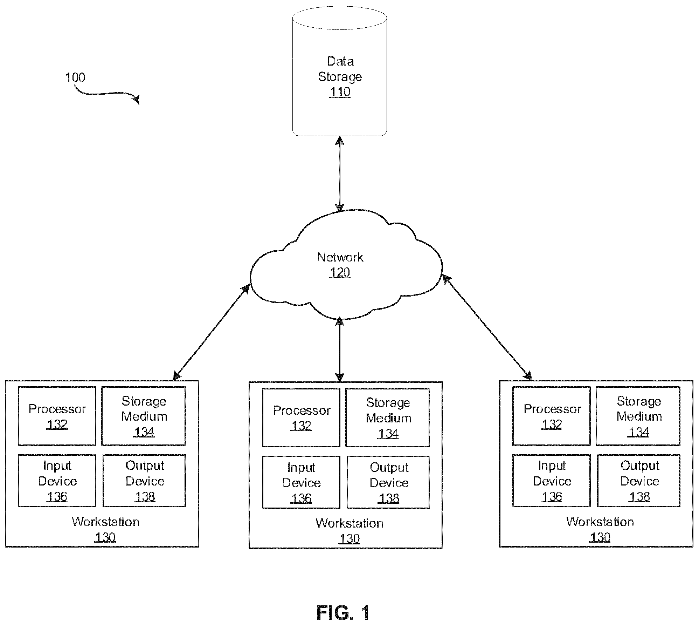

FIG. 1 is a block diagram of an exemplary narcotic tracking system 100 that is operable to track dispensed and returned narcotics, in accordance with various embodiments. Referring to FIG. 1, there is shown an narcotic tracking system 100 comprising at least one data storage 110, at least one network 120, and at least one workstation 130.. While three workstations 130 are illustrated in system 100, a larger or smaller number of workstations 130 can be used in accordance with embodiments of the presently described technology. In addition, while one data storage 110 is illustrated in system 100, system 100 can include more than one data storage 110. For example, each of a plurality of entities (such as remote data storage facilities, hospitals or clinics) can each include one or more data stores 110 in communication with one or more workstations 130 via one or more networks 120. Furthermore, while one network 120 is illustrated in system 100, system 100 may include more than one network 120.

Data storage 110 can be implemented using a variety of devices for storing electronic information such as a file transfer protocol ("FTP") server, for example. Data storage 110 includes electronic data. For example, data storage 110 can store narcotic bag electronic records, anesthesia narcotic records and/or other information, electronic medical records, patient orders, etc., for pharmacies, anesthesia providers, patients, and the like. Data storage 110 may include and/or be in communication with one or more clinical information systems, for example.

Communication between workstations 130, workstations 130 and data storage 110, and/or a plurality of data stores 110 can be via any one or more types of known networks 120 including a local area network ("LAN"), a wide area network ("WAN"), an intranet, or a global network (for example, Internet). Any two of workstations 130 and data stores 110 can be coupled to one another through multiple networks (for example, intranet and Internet) so that not all components of system 100 are required to be coupled to one another through the same network.

Any workstations 130 and/or data stores 110 can be connected to a network 120 or one another in a wired or wireless fashion. In an example embodiment, workstations 130 and data store 110 communicate via the Internet and each workstation 130 executes a user interface application to directly connect to data store 110. In another embodiment, workstation 130 can execute a web browser to contact data store 110. Alternatively, workstation 130 can be implemented using a device programmed primarily for accessing data store 110.

Data storage 110 can be implemented using a server operating in response to a computer program stored in a storage medium accessible by the server. Data storage 110 can operate as a network server (often referred to as a web server) to communicate with workstations 130. Data storage 110 can handle sending and receiving information to and from workstations 130 and can perform associated tasks. Data storage 110 can also include a firewall to prevent unauthorized access and enforce any limitations on authorized access. For instance, an administrator can have access to the entire system 100 and have authority to modify portions of system 100 and a staff member can only have access to view a subset of the data stored at data store 110. In an example embodiment, the administrator has the ability to add new users, delete users and edit user privileges. The firewall can be implemented using conventional hardware and/or software.

Data store 110 can also operate as an application server. Data store 110 can execute one or more application programs to provide access to the data repository located on data store 110. Processing can be shared by data store 110 and workstations 130 by providing an application (for example, a java applet). Alternatively, data store 110 can include a stand-alone software application for performing a portion of the processing described herein. It is to be understood that separate servers may be used to implement the network server functions and the application server functions. Alternatively, the network server, firewall and the application server can be implemented by a single server executing computer programs to perform the requisite functions.

Still referring to FIG. 1, one or more workstations 130 can be in communication with at least one other workstation 130 and/or at least one data storage 110. Workstations 130 can be directly attached to one or more data stores 110 and/or communicate with data store(s) 110 via one or more networks 120. Workstations 130 can be located in a single physical location or in a plurality of locations. Workstations 130 can be connected to and communicate via the one or more networks 120 by any suitable combination of wired or wireless data communication links. Each workstation 130 can be implemented using a specialized or general-purpose computer executing a computer program for carrying out the processes described herein. Workstations 130 can be personal computers or host attached terminals, for example. Workstations 130 may be any of, for example, a desktop computer, a laptop computer, a notebook computer, a netbook computer, a tablet computer, a mobile phone, or any other electronic device having capabilities suitable for accessing the network 120. If workstations 130 are personal computers, the processing described herein can be shared by one or more data stores 110 and a workstation 130 by providing an applet to workstation 130, for example.

In various embodiments, the workstation(s) 130 includes a processor 132, storage medium 134, input device(s) 136, and output device(s) 138, among other things. Components of the workstation(s) 130 may be implemented in software, hardware, firmware, and/or the like. The various components of the workstation(s) 130 may be communicatively linked. Components of the workstation(s) 130 may be implemented separately and/or integrated in various forms. For example, an output device 138, such as a display, and input device(s) 136 may be integrated as a touchscreen display.

The input device(s) 136 may include any device(s) capable of communicating information from a user and/or at the direction of the user to the processor 16 of the computing device 13, for example. The input device(s) 136 may include button(s), a touchscreen, motion tracking, voice recognition, a mousing device, keyboard, stylus, camera and/or any other device capable of receiving a user directive. In certain embodiments, one or more of the input devices 136 may be integrated into other components, such as a display output device 138, for example. As an example, input device 136 may include a touchscreen display.

The output device 138 may be any device capable of communicating information to a user. For example, the output device 138 may be a display, printer, and/or speaker, among other things. A display output device 138 may include a liquid crystal display, a light emitting diode display, and/or any suitable display. The display output device 138 can be operable to display information, such as information provided by data storage 110, processor 132, and/or the like.

The processor 132 may be one or more central processing units, microprocessors, microcontrollers, and/or the like. The processor 132 may be an integrated component, or may be distributed across various locations, for example. The processor 132 may be capable of executing software applications, receiving input information from user input device(s) 136, and generating an output displayable by a display output device 138, among other things. The processor 132 may be capable of executing any of the method(s) and/or set(s) of instructions discussed below in accordance with the present invention, for example. In certain embodiments, the processor 132 may execute one or more project sharing applications available at server(s) 11 and/or stored at the computing device(s) 13 in response to user inputs received from user input device(s) 14, for example.

In various embodiments, the information provided by the user input device(s) 136 to the processor 132 may be processed by the processor 132 to control creation and storage of narcotic bag electronic records at data store 110. For example, the processor 132 may comprise suitable logic, circuitry, interfaces, and/or code for receiving and/or retrieving user information corresponding with an anesthesia provider, receiving and/or retrieving information regarding narcotics being checked-out, and may generate a narcotic bag electronic record based on the anesthesia provider user information and the narcotic check-out information for storage at data store 110. As another example, the processor 132 may comprise suitable logic, circuitry, interfaces, and/or code for retrieving narcotic bag case data from the narcotic bag electronic record in response to a selection of a narcotic bag to be returned, retrieving narcotic administration data from anesthesia narcotic records based on the anesthesia provider associated with the narcotic bag to be returned, determining expected narcotics to be returned based on the narcotic bag case data and the narcotic administration data, and updating narcotic bag electronic records with check-in information and/or discrepancy information.

In an exemplary embodiment, the processor 132 may comprise suitable logic, circuitry, interfaces, and/or code for creating and/or updating a discrepancy worklist with active discrepancies to be resolved. In a representative embodiment, the processor 132 may comprise suitable logic, circuitry, interfaces, and/or code for compiling narcotic administration information and waste information by provider and/or by organization. The compiled narcotic administration information and waste information may be converted by the processor 132 to a common measurement for analysis. For example, amounts of opioid narcotics, such as Fentanyl, Morphine, Hydromorphone, Oxycodone, Midazolam, and the like, may be converted by the processor 132 into a common measurement, such as a Morphine Milligram Equivalent. The converted narcotic administration information may be normalized by the processor 132 to a per minute basis. The processor 132 may comprise suitable logic, circuitry, interfaces, and/or code for performing diversion analytics and waste analytics for presentation at a display output device 138 and/or storage at the data store 110, storage medium 134, and or any suitable data storage device.

Still referring to FIG. 1, the storage medium 134 may be one or more computer-readable memories, for example, such as a hard disk, floppy disk, CD, CD-ROM, DVD, compact storage, flash memory, random access memory, read-only memory, electrically erasable and programmable read-only memory and/or any suitable memory. The storage medium 134 may include databases, libraries, sets of information, or other storage accessed by and/or incorporated with the processor 132, for example. The storage medium 134 may be able to store data temporarily or permanently, for example. The storage medium 134 may be capable of storing data generated by the processor 132 and/or instructions readable by the processor 132, among other things. The storage medium 134 can be included in workstations 130 or physically remote from workstations 130. For example, storage medium 134 can be accessible by workstations 130 through a wired or wireless network connection. In various embodiments, the storage medium 134 stores one or more sets of instructions for a computer. The set(s) of instructions may include one or more routines capable of being run or performed by workstations 130. The set(s) of instructions can be embodied in one or more software applications or in computer code. For example, the set(s) of instructions may include instructions for creating narcotic bag electronic records, retrieving narcotic bag case data and information related to administered narcotics from medical records, determining expected narcotics to be returned, updating narcotic bag electronic records with check-in information and/or discrepancies, maintaining a discrepancy worklist, compiling narcotic administration information and waste information by provider and/or by organization, converting narcotics to a common measurement for analysis, normalizing narcotic administration data to a per minute basis, performing diversion analytics, performing waste analytics, and the like.

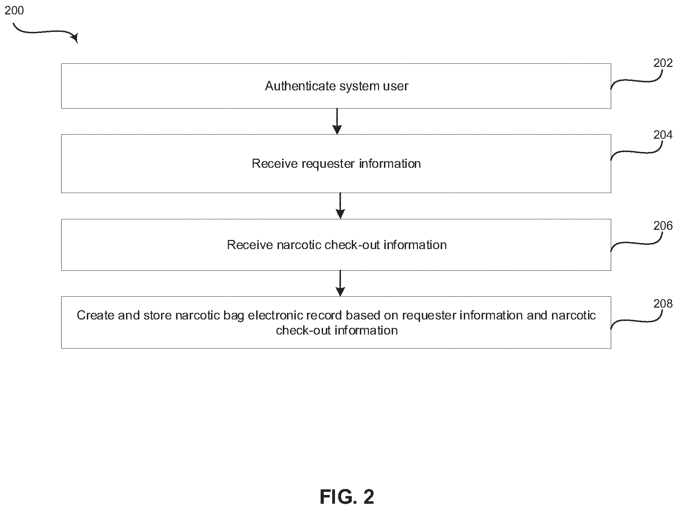

FIG. 2 is a flow chart 200 illustrating exemplary steps 202-208 that may be utilized for checking out narcotics by a narcotic tracking system 100, in accordance with various embodiments. Referring to FIG. 2, there is shown a flow chart 200 comprising exemplary steps 202 through 208. Certain embodiments may omit one or more of the steps, and/or perform the steps in a different order than the order listed, and/or combine certain of the steps discussed below. For example, some steps may not be performed in certain embodiments. As a further example, certain steps may be performed in a different temporal order, including simultaneously, than listed below.

At step 202, narcotic tracking system 100 authenticates a system user. For example, a pharmacy technician at a hospital pharmacy may log-in to the narcotic tracking system 100 to check-out narcotics to anesthesia providers. The narcotic system 100 may comprise a workstation 130 having an input device 136 to receive log-in information from the pharmacy technician. As an example, the narcotics tracking system 100 may comprise a keyboard, keypad, touchscreen, bar code reader, radio-frequency identification (RFID) reader, near field communication (NFC) reader, biometric reader, and/or any suitable input device 136 configured to receive user credentials for logging into the tracking system 100. A data store 110 or processor 132 of the tracking system 100 may be configured to compare the received log-in information with log-in information stored at the data store 110 or any suitable data storage medium of the narcotic tracking system 100 to authenticate the user log-in credentials and grant access to the narcotic tracking system 100.

At step 204, a processor 132 of a workstation 130 of the narcotics tracking system 100 may receive requester information. For example, the pharmacy technician authenticated at step 202 may provide information via the input device 136 to the processor 132 of the workstation 130 regarding the anesthesia provider requesting narcotics. The requester information may include a name, identification number, employee badge bar code, and/or any suitable information to identify the anesthesia provider requesting the narcotics. The requester information may be associated with anesthesia narcotic records and/or medical records of one or more patients the anesthesia provider is scheduled to administer narcotics.

At step 206, the processor 132 of the workstation 130 may receive narcotic check-out information. For example, the pharmacy technician may employ the input device 136 of the workstation 130 to scan or otherwise select a narcotic bag to check-out to the anesthesia provider. The narcotic bag may have default types and/or quantities of narcotics. Additionally and/or alternatively, the input device 136 may scan and/or otherwise select individual types and quantities of narcotics to provide in the narcotics bag. As an example, the pharmacy technician may scan and/or select additional types and/or quantities of narcotics to include in the narcotics bag.

At step 208, the processor 132 of the workstation 130 creates and stores a narcotic bag electronic record based on the requester information and narcotic check-out information. For example, the processor 132 may generate a record that includes the name, identification, employee badge bar code, and/or any suitable information identifying the anesthesia provider checking-out the narcotics bag. The processor 132 may enter and/or otherwise associate the selected narcotics bag and/or any additional narcotics included with the narcotics bag into the narcotic bag electronic record. The processor 132 may store the narcotic bag electronic record at data store 110 and/or any suitable data storage medium of the narcotic tracking system 100.

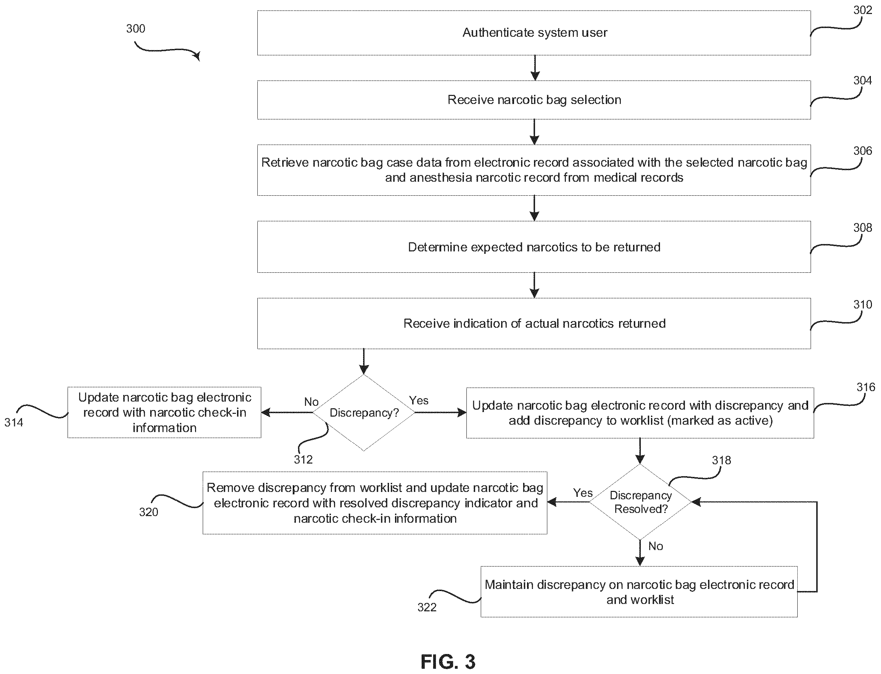

FIG. 3 is a flow chart 300 illustrating exemplary steps 302-322 that may be utilized for checking in narcotics by a narcotic tracking system 100, in accordance with various embodiments. Referring to FIG. 3, there is shown a flow chart 300 comprising exemplary steps 302 through 322. Certain embodiments may omit one or more of the steps, and/or perform the steps in a different order than the order listed, and/or combine certain of the steps discussed below. For example, some steps may not be performed in certain embodiments. As a further example, certain steps may be performed in a different temporal order, including simultaneously, than listed below.

At step 302, narcotic tracking system 100 authenticates a system user. For example, a pharmacy technician at a hospital pharmacy may log-in to the narcotic tracking system 100 to check-in narcotics from anesthesia providers. The narcotic system 100 may comprise a workstation 130 having an input device 136 to receive log-in information from the pharmacy technician. As an example, the narcotics tracking system 100 may comprise a keyboard, keypad, touchscreen, bar code reader, radio-frequency identification (RFID) reader, near field communication (NFC) reader, biometric reader, and/or any suitable input device 136 configured to receive user credentials for logging into the tracking system 100. A data store 110 or processor 132 of the tracking system 100 may be configured to compare the received log-in information with log-in information stored at the data store 110 or any suitable data storage medium of the narcotic tracking system 100 to authenticate the user log-in credentials and grant access to the narcotic tracking system 100.

At step 304, a processor 132 of a workstation 130 of the narcotics tracking system 100 may receive a narcotic bag selection. For example, the pharmacy technician authenticated at step 302 may provide information via the input device 136 to the processor 132 of the workstation 130 regarding a narcotics bag previously checked-out by an anesthesia provider. The narcotics bag may be associated with a bag number, anesthesia provider name, anesthesia provider identification number, narcotics bag bar code, anesthesia provider employee badge bar code, and/or any suitable information to identify the narcotics bag previously checked-out to the anesthesia provider. As an example, a pharmacy technician my employ a bar code scanning input device 136 to scan a bar code on a returned narcotics bag or an anesthesia provider employee badge. As another example, a list of checked-out bags may be provided at a display output device 138 for selection by the pharmacy technician via the input device 136. The narcotics bag being returned and/or the anesthesia provider returning the bag may be associated with a narcotics bag electronic record stored at data store 110 or any suitable data storage medium of the narcotics tracking system 100.

At step 306, the processor 132 of the workstation 130 may retrieve narcotic bag case data from an electronic record associated with the selected narcotic bag and one or more anesthesia narcotic records from medical records. For example, the processor 132 may retrieve narcotic bag case data from the narcotic bag electronic record in response to the bag selection at step 304. The narcotic bag case data may include information regarding the anesthesia provider that checked out the bag and the types and quantities of narcotics that were checked out. The processor 132 may retrieve anesthesia narcotic records from electronic medical records stored at data store 110 and/or any suitable data storage medium based on the anesthesia provider information associated with the narcotic bag electronic record. The anesthesia narcotic records may include information regarding the narcotics administered to patients by the anesthesia provider.

At step 308, the processor 132 of the workstation 130 may determine expected narcotics to be returned. For example, the processor 132 may be operable to calculate the expected narcotics to be returned based on an amount of each narcotic checked-out as indicated in the retrieved narcotic bag case data compared to an amount of each narcotic used as indicated in the retrieved anesthesia narcotic records.

At step 310, the processor 132 of the workstation 130 may receive an indication of actual narcotics returned. For example, the pharmacy technician may employ the input device 136 of the workstation to enter the amount of each narcotic returned in the narcotics bag. The processor 132 receives the entered amount of each narcotic via the input device 136. In various embodiments, the pharmacy technician may test the narcotics with a refractometer input device 136 to confirm the identity of each of the returned narcotics. The test results from the refractometer input device 136 may be provided to the processor 132.

At step 312, the processor 132 of the workstation 130 determines whether there is a discrepancy with the returned narcotics. For example, the processor 132 may calculate whether the actual narcotics returned at step 310 corresponds with the expected narcotics to be returned determined at step 308. The processor 132 may determine whether tested returned narcotics correspond with the expected narcotics to be returned. If a discrepancy is present as determined by the processor 132, the process proceeds to step 316. If the processor 132 determines that no discrepancy is present, the process proceeds to step 314.

At step 314, the processor 132 of the workstation 130 updates the narcotic bag electronic record with narcotic check-in information. For example, if the processor 132 determines that no discrepancy is present at step 312, the processor 132 may be operable to update the narcotic bag electronic record with the amounts of each narcotic returned. In various embodiments, the processor 132 may enter narcotic use information into the narcotic bag electronic record based on the anesthesia narcotic records retrieved at step 306.

At step 316, the processor 132 of the workstation 130 updates the narcotic bag electronic record with the detected discrepancy and adds the discrepancy (marked as active) to a discrepancy worklist. For example, if the processor 132 determines that one or more discrepancies are present at step 312, the processor 132 may be operable to update the narcotic bag electronic record with the noted discrepancy. The processor 132 may include any notes or additional information related to the discrepancy provided by the pharmacy technician via the input device 136 to the narcotic bag electronic record. The processor 132 may be configured to add the discrepancy to a discrepancy worklist stored at data store 110 and/or any suitable data storage medium of the narcotic tracking system 100 and to mark the discrepancy as active until resolved. The discrepancy worklist may be a list of discrepancy that have not been resolved. The discrepancy worklist may be accessible from the data store 110 by anesthesia supervisors or other medical professionals that may investigate and resolve the active discrepancies.

At step 318, the processor 132 of the workstation 130 determines whether a discrepancy with returned narcotics provided in the discrepancy worklist has been resolved. For example, the processor 132 may receive an input from a user authorized to investigate discrepancies identifying a discrepancy in the worklist that has been resolved. If the processor 132 receives an input that a discrepancy has been resolved, the process proceeds to step 320. If the processor 132 does not receive an input that the discrepancy has been resolved, the process proceeds to step 322.

At step 320, the processor 132 of the workstation 130 removes the discrepancy from the worklist and updates the narcotic bag electronic record with a resolved discrepancy indicator and narcotic check-in information. For example, if the processor 132 determines that the discrepancy is resolved at step 318, the processor 132 may be operable to update the narcotic bag electronic record with the amounts of each narcotic returned and an indication that the discrepancy has been resolved. The indication that the discrepancy has been resolved may include notes or other information describing the circumstances of the resolution. In various embodiments, the processor 132 may enter narcotic use information into the narcotic bag electronic record based on the anesthesia narcotic records retrieved at step 306 as modified (if necessary) by the discrepancy resolution at step 318.

At step 322, the processor 132 of the workstation 130 maintains the discrepancy on the narcotic bag electronic record and worklist. For example, if the processor 132 determines that the discrepancy has not been resolved at step 318, the processor 132 may be operable to maintain the discrepancy on the narcotic bag electronic record and the discrepancy remains active on the discrepancy worklist. The process 300 returns to step 318 until the discrepancy has been resolved. The process ends at step 314 or step 320 when the narcotics have been returned, any discrepancies have been resolved, and the narcotic bag electronic record (and the discrepancy worklist, if applicable) have been updated.

In an exemplary embodiment, the processor 132 of the workstation 130 may be configured to analyze narcotic bag electronic records across anesthesia providers, procedure categories, and/or medical organizations to provide statistics regarding administered and/or wasted narcotics by provider, by procedure type, by organization, and the like. For example, the processor 132 of the workstation 130 may retrieve narcotic bag electronic records corresponding to narcotic administration by an anesthesia provider, by a plurality of anesthesia providers, by procedure category (e.g., cardiac/thoracic surgery, general surgery, neuro surgery, gynecology surgery, etc.), by medical group, by hospital, and/or any suitable categories or groups. The processor 132 may comprise suitable logic, circuitry, interfaces, and/or code for converting narcotics to a common measurement. For example, the processor 132 may perform calculations to convert opioid narcotics, such as Fentanyl, Morphine, Hydromorphone, Oxycodone, Midazolam, and the like to a common measurement, such as a Morphine Milligram Equivalent, to allow a comparison of narcotics strength across providers that may be administering different narcotics to patients. In various embodiments, the processor 132 may be configured to perform analytics against peers of an anesthesia provider to determine a standard deviation of narcotic administration to determine when a provider may fall outside of 1 standard deviation, indicating that diversion may be occurring or that administration practices by that provider should be reviewed to ensure compliance with organization polices and best practices. The processor 132 may perform the analysis for each anesthesia provider on data from all cases and a subset of the most recent cases (e.g., last 100 cases) to detect trends and outliers early and prevent changes in administration trends from being hidden in an average of a large number of cases. The processor 132 may be configured to normalize the narcotic administration data to a per minute basis. In a representative embodiment, the processor 132 may calculate a complete data set of administered narcotics and returned (i.e., wasted) narcotics to present analysis of average dose administered and average amount wasted per case for operational purposes, such as adjusting the narcotics provided in checked-out narcotic bags.

Aspects of the present disclosure provide a method 200, 300 and system 100 for tracking dispensed and returned narcotics. In accordance with various embodiments, the method 200, 300 may comprise receiving 304, by at least one processor 132, a selection of one of a plurality of narcotic bags. Each of the plurality of narcotic bags may be associated with one of a plurality of narcotic bag electronic records. The method 200, 300 may comprise retrieving 306, by the at least one processor, narcotic bag case data from the one of the plurality of narcotic bag electronic records associated with the selected one of the plurality of narcotic bags. The method 200, 300 may comprise retrieving 306, by the at least one processor 132, an anesthesia narcotic record from electronically stored medical records. The method 200, 300 may comprise automatically determining 308, by the at least one processor 132, expected narcotics to be returned based on the narcotic bag case data and the anesthesia narcotic record. The method 200, 300 may comprise receiving 310, by the at least one processor 132, an indication of actual narcotics returned. The method 200, 300 may comprise automatically determining 312, by the at least one processor 132, whether a discrepancy exists based on the expected narcotics to be returned and the actual narcotics returned. The method 200, 300 may comprise modifying 314, 316, by the at least one processor 132, the one of the plurality of narcotic bag electronic records to include at least one of the actual narcotics returned and a discrepancy indicator.

In an exemplary embodiment, the narcotic bag electronic record and the anesthesia narcotic record are retrieved via at least one network 120 from at least one data store 110. In a representative embodiment, each of the expected narcotics to be returned and the actual narcotics returned comprises at least one narcotic type and a narcotic amount corresponding to each of the at least one narcotic type. In certain embodiments, the method 200, 300 may comprise automatically determining 312, by the at least one processor 132, the discrepancy exists. The method 200, 300 may comprise modifying 316, by the at least one processor 132, the one of the plurality of narcotic bag electronic records to include the discrepancy indicator. The method 200, 300 may comprise modifying 316, by the at least one processor 132, a discrepancy worklist to include the discrepancy. In various embodiments, the method 200, 300 may comprise receiving 318, by the at least one processor 132, an indication that the discrepancy is resolved. The method 200, 300 may comprise modifying 320, by the at least one processor 132, the one of the plurality of narcotic bag electronic records to include a resolved discrepancy indicator. The method 200, 300 may comprise modifying 320, by the at least one processor 132, the discrepancy worklist to remove the discrepancy.

In certain embodiments, the method 200, 300 may comprise receiving 202, by the at least one processor 132, anesthesia provider identifying information. The method 200, 300 may comprise receiving 206, by the at least one processor 132, narcotic check-out information comprising the one of the plurality of narcotic bags, at least one narcotic type being checked-out and a narcotic amount corresponding to each of the at least one narcotic type being checked-out. The method 200, 300 may comprise creating 208, by the at least one processor 132, the one of the plurality of narcotic bag electronic records based on the anesthesia provider identifying information and the narcotic check-out information. The method 200, 300 may comprise storing 208, by the at least one processor 132, the one of the plurality of narcotic bag electronic records at one or more data stores 110 over at least one network 120. In a representative embodiment, the method 200, 300 may comprise retrieving, by the at least one processor 132, a plurality of the plurality of narcotic bag electronic records. The method 200, 300 may comprise converting, by the at least one processor 132, amounts of different narcotics to a common measurement. The method 200, 300 may comprise analyzing, by the at least one processor 132, a plurality of anesthesia providers based on the retrieved plurality of the plurality of narcotic bag electronic records to determine a standard deviation of narcotic administration. The standard deviation of narcotic administration for the plurality of anesthesia providers may be presented at a display system 138. Additionally and/or alternatively, the method 200, 300 may comprise analyzing, by the at least one processor 132, one or both of a total amount of administered narcotics and a total amount of returned narcotics to calculate one or both of an average administered dose and an average amount wasted. One or both of the average administered dose and the average amount wasted may be presented at the display system 138.

Various embodiments provide a system 100 for tracking dispensed and returned narcotics. The system 100 may comprise at least one input device 136, at least one data store 110, and at least one processor 132. The at least one input device 136 may be operable to provide a selection of one of a plurality of narcotic bags. Each of the plurality of narcotic bags may be associated with one of a plurality of narcotic bag electronic records. The at least one input device 136 may be operable to provide an indication of actual narcotics returned. The at least one data store 110 may be configured to store the plurality of narcotic bag electronic records. The at least one data store 110 may be configured to store medical records comprising an anesthesia narcotic record. The at least one processor 132 may be configured to receive the selection of the one of the plurality of narcotic bags from the at least one input device 136. The at least one processor 132 may be configured to retrieve narcotic bag case data from the one of the plurality of narcotic bag electronic records associated with the selected one of the plurality of narcotic bags. The at least one processor 132 may be configured to retrieve the anesthesia narcotic record from the medical records. The at least one processor 132 may be configured to automatically determine expected narcotics to be returned based on the narcotic bag case data and the anesthesia narcotic record. The at least one processor 132 may be configured to receive the indication of the actual narcotics returned from the at least one input device 136. The at least one processor 132 may be configured to automatically determine whether a discrepancy exists based on the expected narcotics to be returned and the actual narcotics returned. The at least one processor 132 may be configured to modify the one of the plurality of narcotic bag electronic records stored at the at least one data store 110 to include at least one of the actual narcotics returned and a discrepancy indicator.

In a representative embodiment, the system 100 may comprise at least one network 120 communicatively coupling the at least one processor 132 and the at least on data store 110. In various embodiments, the at least one processor 132 may be configured to automatically determine the discrepancy exists. The at least one processor 132 may be configured to modify the one of the plurality of narcotic bag electronic records to include the discrepancy indicator. The at least one processor 132 may be configured to modify a discrepancy worklist to include the discrepancy. In certain embodiments, the at least one input device 136 may be operable to provide an indication that the discrepancy is resolved. The at least one processor 132 may be configured to receive the indication that the discrepancy is resolved from the at least one input device 136. The at least one processor 132 may be configured to modify the one of the plurality of narcotic bag electronic records to include a resolved discrepancy indicator. The at least one processor 132 may be configured to modify the discrepancy worklist to remove the discrepancy.

In various embodiments, the at least one input device 136 may be operable to provide anesthesia provider identifying information. The at least one input device 136 may be operable to provide narcotic check-out information comprising the one of the plurality of narcotic bags, at least one narcotic type being checked-out, and a narcotic amount corresponding to each of the at least one narcotic type being checked-out. The at least one processor 132 may be configured to receive the anesthesia provider identifying information from the at least one input device 136. The at least one processor 132 may be configured to receive the narcotic check-out information from the at least one input device 136. The at least one processor 132 may be configured to create the one of the plurality of narcotic bag electronic records based on the anesthesia provider identifying information and the narcotic check-out information. The at least one processor 132 may be configured to store the one of the plurality of narcotic bag electronic records at the at least one data store 110. In an exemplary embodiment, the system 100 may comprise a display system 138. The at least one processor 132 may be configured to retrieve a plurality of the plurality of narcotic bag electronic records from the at least one data store 110. The at least one processor 132 may be configured to convert amounts of different narcotics to a common measurement. The at least one processor 132 may be configured to analyze a plurality of anesthesia providers based on the retrieved plurality of the plurality of narcotic bag electronic records to determine a standard deviation of narcotic administration. The standard deviation of narcotic administration for the plurality of anesthesia providers may be presented at the display system 138. Additionally and/or alternatively, the at least one processor 132 may be configured to analyze one or both of a total amount of administered narcotics and a total amount of returned narcotics to calculate one or both of an average administered dose and an average amount wasted. One or both of the average administered dose and the average amount wasted may be presented at the display system 138.

Certain embodiments provide a non-transitory computer readable medium having stored thereon, a computer program having at least one code section. The at least one code section is executable by a machine for causing the machine to perform steps 200, 300. The steps 200, 300 may comprise receiving 304 a selection of one of a plurality of narcotic bags. Each of the plurality of narcotic bags may be associated with one of a plurality of narcotic bag electronic records. The steps 200, 300 may comprise retrieving 306 narcotic bag case data from the one of the plurality of narcotic bag electronic records associated with the selected one of the plurality of narcotic bags. The steps 200, 300 may comprise retrieving 306 an anesthesia narcotic record from electronically stored medical records. The steps 200, 300 may comprise automatically determining 308 expected narcotics to be returned based on the narcotic bag case data and the anesthesia narcotic record. The steps 200, 300 may comprise receiving 310 an indication of actual narcotics returned. The steps 200, 300 may comprise automatically determining 312 whether a discrepancy exists based on the expected narcotics to be returned and the actual narcotics returned. The steps 200, 300 may comprise modifying 314, 316 the one of the plurality of narcotic bag electronic records to include at least one of the actual narcotics returned and a discrepancy indicator.

In an exemplary embodiment, the narcotic bag electronic record and the anesthesia narcotic record are retrieved via at least one network 120 from at least one data store 110. In various embodiments, each of the expected narcotics to be returned and the actual narcotics returned comprises at least one narcotic type and a narcotic amount corresponding to each of the at least one narcotic type. In a representative embodiment, the steps 200, 300 may comprise automatically determining 312 the discrepancy exists. The steps 200, 300 may comprise modifying 316 the one of the plurality of narcotic bag electronic records to include the discrepancy indicator. The steps 200, 300 may comprise modifying 316 a discrepancy worklist to include the discrepancy. In certain embodiments, the steps 200, 300 may comprise receiving 318 an indication that the discrepancy is resolved. The steps 200, 300 may comprise modifying 320 the one of the plurality of narcotic bag electronic records to include a resolved discrepancy indicator. The steps 200, 300 may comprise modifying 320 the discrepancy worklist to remove the discrepancy.

In a representative embodiment, the steps 200, 300 may comprise receiving 204 anesthesia provider identifying information. The steps 200, 300 may comprise receiving 206 narcotic check-out information comprising the one of the plurality of narcotic bags, at least one narcotic type being checked-out, and a narcotic amount corresponding to each of the at least one narcotic type being checked-out. The steps 200, 300 may comprise creating 208 the one of the plurality of narcotic bag electronic records based on the anesthesia provider identifying information and the narcotic check-out information. The steps 200, 300 may comprise storing 208 the one of the plurality of narcotic bag electronic records at one or more data stores 110 over at least one network 120. In an exemplary embodiment, the steps 200, 300 may comprise retrieving a plurality of the plurality of narcotic bag electronic records. The steps 200, 300 may comprise converting amounts of different narcotics to a common measurement. The steps 200, 300 may comprise analyzing a plurality of anesthesia providers based on the retrieved plurality of the plurality of narcotic bag electronic records to determine a standard deviation of narcotic administration. The standard deviation of narcotic administration for the plurality of anesthesia providers may be presented at a display system 138. The steps 200, 300 may comprise analyzing one or both of a total amount of administered narcotics and a total amount of returned narcotics to calculate one or both of an average administered dose and an average amount wasted. One or both of the average administered dose and the average amount wasted may be presented at the display system 138.

As utilized herein the term "circuitry" refers to physical electronic components (i.e. hardware) and any software and/or firmware ("code") which may configure the hardware, be executed by the hardware, and or otherwise be associated with the hardware. As used herein, for example, a particular processor and memory may comprise a first "circuit" when executing a first one or more lines of code and may comprise a second "circuit" when executing a second one or more lines of code. As utilized herein, "and/or" means any one or more of the items in the list joined by "and/or". As an example, "x and/or y" means any element of the three-element set {(x), (y), (x, y)}. As another example, "x, y, and/or z" means any element of the seven-element set {(x), (y), (z), (x, y), (x, z), (y, z), (x, y, z)}. As utilized herein, the term "exemplary" means serving as a non-limiting example, instance, or illustration. As utilized herein, the terms "e.g.," and "for example" set off lists of one or more non-limiting examples, instances, or illustrations. As utilized herein, circuitry is "operable" and/or "configured" to perform a function whenever the circuitry comprises the necessary hardware and code (if any is necessary) to perform the function, regardless of whether performance of the function is disabled, or not enabled, by some user-configurable setting.

Other embodiments may provide a computer readable device and/or a non-transitory computer readable medium, and/or a machine readable device and/or a non-transitory machine readable medium, having stored thereon, a machine code and/or a computer program having at least one code section executable by a machine and/or a computer, thereby causing the machine and/or computer to perform the steps as described herein for tracking dispensed and returned narcotics.

Accordingly, the present disclosure may be realized in hardware, software, or a combination of hardware and software. The present disclosure may be realized in a centralized fashion in at least one computer system, or in a distributed fashion where different elements are spread across several interconnected computer systems. Any kind of computer system or other apparatus adapted for carrying out the methods described herein is suited.

Various embodiments may also be embedded in a computer program product, which comprises all the features enabling the implementation of the methods described herein, and which when loaded in a computer system is able to carry out these methods. Computer program in the present context means any expression, in any language, code or notation, of a set of instructions intended to cause a system having an information processing capability to perform a particular function either directly or after either or both of the following: a) conversion to another language, code or notation; b) reproduction in a different material form.

While the present disclosure has been described with reference to certain embodiments, it will be understood by those skilled in the art that various changes may be made and equivalents may be substituted without departing from the scope of the present disclosure. In addition, many modifications may be made to adapt a particular situation or material to the teachings of the present disclosure without departing from its scope. Therefore, it is intended that the present disclosure not be limited to the particular embodiment disclosed, but that the present disclosure will include all embodiments falling within the scope of the appended claims.

## Claims

1. A method (200, 300) comprising:
receiving (304), by at least one processor, a selection of one of a plurality of narcotic bags, each of the plurality of narcotic bags associated with one of a plurality of narcotic bag electronic records;
retrieving (306), by the at least one processor, narcotic bag case data from the one of the plurality of narcotic bag electronic records associated with the selected one of the plurality of narcotic bags;
retrieving (306), by the at least one processor, an anesthesia narcotic record from electronically stored medical records;
automatically determining (308), by the at least one processor, expected narcotics to be returned based on the narcotic bag case data and the anesthesia narcotic record;
receiving (310), by the at least one processor, an indication of actual narcotics returned;
automatically determining (312), by the at least one processor, whether a discrepancy exists based on the expected narcotics to be returned and the actual narcotics returned; and
modifying (316), by the at least one processor, the one of the plurality of narcotic bag electronic records to include at least one of the actual narcotics returned and a discrepancy indicator.

2. The method (200, 300) of claim 1, wherein the narcotic bag electronic record and the anesthesia narcotic record are retrieved via at least one network (120) from at least one data store (110).

3. The method (200, 300) of claim 1, wherein each of the expected narcotics to be returned and the actual narcotics returned comprises at least one narcotic type and a narcotic amount corresponding to each of the at least one narcotic type.

4. The method (200, 300) of claim 1, comprising:
automatically determining (312), by the at least one processor, the discrepancy exists;
modifying (316), by the at least one processor, the one of the plurality of narcotic bag electronic records to include the discrepancy indicator; and
modifying (316), by the at least one processor, a discrepancy worklist to include the discrepancy.

5. The method (200, 300) of claim 4, comprising:
receiving, by the at least one processor, an indication that the discrepancy is resolved;
modifying, by the at least one processor, the one of the plurality of narcotic bag electronic records to include a resolved discrepancy indicator; and
modifying (320), by the at least one processor, the discrepancy worklist to remove the discrepancy.

6. The method (200, 300) of claim 1, comprising:
receiving (204), by the at least one processor, anesthesia provider identifying information;
receiving (206), by the at least one processor, narcotic check-out information comprising the one of the plurality of narcotic bags, at least one narcotic type being checked-out, and a narcotic amount corresponding to each of the at least one narcotic type being checked-out;
creating (208), by the at least one processor, the one of the plurality of narcotic bag electronic records based on the anesthesia provider identifying information and the narcotic check-out information; and
storing (208), by the at least one processor, the one of the plurality of narcotic bag electronic records at one or more data stores over at least one network.

7. The method (200, 300) of claim 1, comprising:
retrieving, by the at least one processor, a plurality of the plurality of narcotic bag electronic records;
converting, by the at least one processor, amounts of different narcotics to a common measurement; and
one or both of:
analyzing, by the at least one processor, a plurality of anesthesia providers based on the retrieved plurality of the plurality of narcotic bag electronic records to determine a standard deviation of narcotic administration, wherein the standard deviation of narcotic administration for the plurality of anesthesia providers is presented at a display system; or
analyzing, by the at least one processor, one or both of a total amount of administered narcotics and a total amount of returned narcotics to calculate one or both of an average administered dose and an average amount wasted, wherein one or both of the average administered dose and the average amount wasted is presented at the display system.

8. A system (100) comprising:
at least one input device (136) operable to:
provide a selection of one of a plurality of narcotic bags, each of the plurality of narcotic bags associated with one of a plurality of narcotic bag electronic records; and
provide an indication of actual narcotics returned;
at least one data store (110) configured to:
store the plurality of narcotic bag electronic records; and
store medical records comprising an anesthesia narcotic record; and at least one processor (132) configured to:
receive the selection of the one of the plurality of narcotic bags from the at least one input device;
retrieve narcotic bag case data from the one of the plurality of narcotic bag electronic records associated with the selected one of the plurality of narcotic bags;
retrieve the anesthesia narcotic record from the medical records;
automatically determine expected narcotics to be returned based on the narcotic bag case data and the anesthesia narcotic record;
receive the indication of the actual narcotics returned from the at least one input device;
automatically determine whether a discrepancy exists based on the expected narcotics to be returned and the actual narcotics returned; and
modify the one of the plurality of narcotic bag electronic records stored at the at least one data store to include at least one of the actual narcotics returned and a discrepancy indicator.

9. The system (100) of claim 8, comprising at least one network communicatively coupling the at least one processor (132) and the at least on data store (110).

10. The system (100) of claim 8, wherein the at least one processor (132) is configured to:
automatically determine the discrepancy exists;
modify the one of the plurality of narcotic bag electronic records to include the discrepancy indicator; and
modify a discrepancy worklist to include the discrepancy.

11. The system (100) of claim 10, wherein the at least one input device (136) is operable to provide an indication that the discrepancy is resolved, and wherein the at least one processor (132) is configured to:
receive the indication that the discrepancy is resolved from the at least one input device;
modify the one of the plurality of narcotic bag electronic records to include a resolved discrepancy indicator; and
modify the discrepancy worklist to remove the discrepancy.

12. The system (100) of claim 8, wherein:
the at least one input device (136) is operable to:
provide anesthesia provider identifying information; and
provide narcotic check-out information comprising the one of the plurality of narcotic bags, at least one narcotic type being checked-out, and a narcotic amount corresponding to each of the at least one narcotic type being checked-out, and
wherein the at least one processor (132) is configured to:
receive the anesthesia provider identifying information from the at least one input device;
receive the narcotic check-out information from the at least one input device;
create the one of the plurality of narcotic bag electronic records based on the anesthesia provider identifying information and the narcotic check-out information; and
store the one of the plurality of narcotic bag electronic records at the at least one data store.

13. The system (100) of claim 8, comprising a display system (138), and wherein the at least one processor (132) is configured to:
retrieve a plurality of the plurality of narcotic bag electronic records from the at least one data store;
convert amounts of different narcotics to a common measurement; and
one or both of:
analyze a plurality of anesthesia providers based on the retrieved plurality of the plurality of narcotic bag electronic records to determine a standard deviation of narcotic administration, wherein the standard deviation of narcotic administration for the plurality of anesthesia providers is presented at the display system; or
analyze one or both of a total amount of administered narcotics and a total amount of returned narcotics to calculate one or both of an average administered dose and an average amount wasted, wherein one or both of the average administered dose and the average amount wasted is presented at the display system.

14. A non-transitory computer readable medium (134) having stored thereon, a computer program having at least one code section, the at least one code section being executable by a machine for causing the machine to perform steps comprising:
receiving (304) a selection of one of a plurality of narcotic bags, each of the plurality of narcotic bags associated with one of a plurality of narcotic bag electronic records;
retrieving (306) narcotic bag case data from the one of the plurality of narcotic bag electronic records associated with the selected one of the plurality of narcotic bags;
retrieving (306) an anesthesia narcotic record from electronically stored medical records;
automatically (308) determining expected narcotics to be returned based on the narcotic bag case data and the anesthesia narcotic record;
receiving (310) an indication of actual narcotics returned;
automatically (312) determining whether a discrepancy exists based on the expected narcotics to be returned and the actual narcotics returned; and
modifying (316) the one of the plurality of narcotic bag electronic records to include at least one of the actual narcotics returned and a discrepancy indicator.

15. The non-transitory computer readable medium (134) of claim 14, wherein the narcotic bag electronic record and the anesthesia narcotic record are retrieved via at least one network (120) from at least one data store (110).
